# EUROPÄISCHE PATENTSCHRIFT

(11) **EP 0 952 134 B1**
(45) Veröffentlichungstag und Bekanntmachung des Hinweises auf die Patenterteilung: **27.11.2002**
(21) Anmeldenummer: 99100467.2
(22) Anmeldetag: 12.01.1999
(51) Int. Cl.: C07C 7/12

(54) **Verfahren zur Trennung von polycyclischen aromatischen Kohlenwasserstoffen (PKA) mittels Flüssigkeitschromatographie**
Process for the separation of polycyclic aromatic hydrocarbons by liquid chromatography
Procédé pour la séparation d'hydrocarbures aromatiques polycycliques par chromatography liquide

(30) Priorität: 21.04.1998 DE 19835604
(43) Veröffentlichungstag der Anmeldung: 27.10.1999
(73) Patentinhaber: Menyes, Ulf, Dr., 17489 Greifswald (DE); Roth, Ulrich, Dr., 17498 Levenhagen (DE)
(72) Erfinder: Meynes, Ulf Dr., 17489 Greifswald (DE); Roth, Ulrich Dr., 17498 Levenhagen (DE); Jira, Thomas Dr., 17498 Potthagen (DE)
(74) Vertreter: Meyerhöfer, Dietmar, Dipl.-Ing.

(56) Entgegenhaltungen:
- CHEMICAL ABSTRACTS, vol. 128, no. 16, 20. April 1998 Columbus, Ohio, US; abstract no. 200300w, S. ZHANG ET AL: "Gas chromatography of calix[4]arene derivatives" Seite 1215; Spalte l; XP002107884 & FENXI KEXUE XUEBAO, Bd. 14, Nr. 1, 1998, Seiten 14-18,
- CHEMICAL ABSTRACTS, vol. 129, no. 23, 7. Dezember 1998 Columbus, Ohio, US; abstract no. 310143a, Z. ZENG ET AL: "Two kinds of calix[4]arene derivatives as capillary gas chromatography stationary phases" Seite 1280; Spalte l; XP002107885 & FENXI HUAXUE, Bd. 26, Nr. 9, 1998, Seiten 1060-1064,
- CHEMICAL ABSTRACTS, vol. 129, no. 23, 7. Dezember 1998 Columbus, Ohio, US; abstract no. 310151b, W. XU ET AL: "Preparation and characterization of p-tert-butyl-calix[6]arene-bonded silica gel stationary phase for high performance liquid chromatography" Seite 1280; Spalte r; XP002107886 & CHROMATOGRAPHIA, Bd. 48, Nr. 3/4, 1998, Seiten 245-250, Wiesbaden DE

## Beschreibung

Die Erfindung betrifft ein Verfahren zur Trennung von polycylischen aromatischen Kohlenwasserstoffen mittels flüssigkeitschromatographischer Methoden.

Da die polycyclischen aromatischen Kohlenwasserstoffe zum Teil carcenogene Wirkung besitzen und damit als Umweltgifte gelten, sind sie im Boden und Wasser zu analysieren.

Bisherige Verfahren bestimmen die polycyclischen aromatischen Kohlenwasserstoffe mit Hilfe von unpolaren Chromatographiephasen, wie die RP- C18-Phasen. Bekannt sind dazu genormte Methoden, die beispielsweise in der Trinkwasserverordnung der Bundesrepublik Deutschland oder in der Untersuchungsvorschrift EPA 610 der Umweltschutzbehörde der Vereinigten Staaten von Amerika festgeschrieben sind.

Weiterhin untersuchten Shaowen, Z et al. : Gas chromatography of calix[4]arene derivatives; veröffentlicht in: Fenxi Kexue Xuebao (1998), 14 (1), S. 14 -18, gaschromatographische Phasen mit zwei verschiedenen Calix[4]arenen und deren Wechselwirkung mit polycyclischen aromatischen Kohlenwasserstoffen und ähnlichen Systemen. Die Autoren gehen davon aus, daß diese mit den Calix[4]arenen Einlagerungsverbindungen eingehen.

Nach Sun, S., Gutsche, D. et al.: Capillary electrokinetic chromatography employing p-(Carboxyethyl)calix[n]arenes as running buffer additives; veröffentlicht in: Anal. Chem. (1997), 69 (3), S. 344-348 und Kalchenko, O. I. et al.: Effect of Octakis(diethoxyphosphoryloxy)-tert-butylcalix[8]arene in mobile phase on the reversed-phase retention behavior of aromatic compounds: host-guest complex formation and stability constants determination; veröffentlicht in: Journal of Chromatographic Science, Vol. 36, May 1998, werden ähnliche Einlagerungsverbindungen beim Einsatz von Calixarenen in der Kapillarelektrophorese (CE) und in der hochauflösenden Flüssigkeitschromatographie (HPLC) beschrieben, wobei die Calixarene verfahrensgemäß als Zusatz zum Laufmittel eingesetzt sind.

Die Aufgabe der Erfindung besteht in einer effektiven Trennung von polycyclischen aromatischen Kohlenwasserstoffen mittels flüssigkeitschromatographischen Methoden.

Erfindungsgemäß wird die Aufgabe dadurch gelöst, daß zur Trennung der polycyclischen aromatischen Kohlenwasserstoffe mittels der Flüssigkeitschromatographie die calixarenmodifizierte stationäre Trennphase zum Einsatz kommt. Hierzu werden die polycyclischen aromatischen Kohlenwasserstoffe in einem geeigneten Lösungsmittel gelöst bzw. können Lösungen von polycyclischen aromatischen Kohlenwasserstoffen in einem flüssigen Medium verwendet werden.

Mit Hilfe eines geeigneten Laufmittels in Form von einem Lösungsmittel und / oder Lösungsmittelgemisch werden die Proben der gelösten polycyclischen aromatischen Kohlenwasserstoffe über die calixarenmodifizierte Trennphase geleitet, wobei sich die polycyclischen aromatischen Kohlenwasserstoffe räumlich voneinander trennen. Anschließend erfolgt dann die Detektion der getrennten Substanzen.

Die Erfindung soll an einem Ausführungsbeispiel näher erläuert werden.

### Beispiel 1:

Mittels einer HPLC-Anlage mit ternären Gradientensystem und UV-Detektor wird ein Gemisch aus Benzol, Naphthalin und Anthracen mit einer Caltrex A I - Säule im Format 25 cm × 4,6 mm aufgetrennt. Als Elutionsmittel wird ein Gemisch aus Acetonitril / Methanol / Wasser im Verhältnis von 1:3:6 (v/v/v) mit einer Fließgeschwindigkeit von 1 ml/min verwendet. Die Detektorwellenlänge beträgt 254 nm.

Folgendes Trennergebnis ist erzielt:

| Substanz | Zeit (min) |
|---|---|
| Benzol | 2,1 |
| Naphthalin | 3,2 |
| Anthracen | 5,4 |

### Beispiel 2:

Mittels einer HPLC-Anlage mit ternären Gradientensystem und UV-Detektor wird ein Gemisch aus Phenanthren, Fluoranthen, Chrysen und Perylen mit einer Caltrex B I - Säule im Format 25 cm × 4,6 mm aufgetrennt. Als Elutionsmittel wird ein Gemisch aus Acetonitril / Methanol / Wasser im Verhältnis von 1:3:6 (v/v/v) mit einer Fließgeschwindigkeit von 1 ml/min verwendet. Die Detektorwellenlänge beträgt 254 nm. Folgendes Trennergebnis ist erzielt:

| Substanz | Zeit (min) |
|---|---|
| Phenanthren | 9,7 |
| Fluoranthen | 10,2 |
| Chrysen | 13,9 |
| Perylen | 18,0 |

Die erfindungsgemäße flüssigkeitschromatografische Trennmethode mit calixarenmodifizierten stationären Trennphasen ist in der Dünn- und Dickschichtchromatographie, Säulenchromatographie wie HPLC, Festphasenextraktion, überkritische Chromatographie wie beispielsweise SCE, Elektrophorese wie beispielsweise CE einsetzbar.

## Patentansprüche

1. Verfahren zur Trennung von polycyclischen aromatischen Kohlenwasserstoffen (PAK) mittels Flüssigkeitschromatographie, **dadurch gekennzeichnet, daß** die Substanzprobe aus verschiedenen PAK in einem Lösungsmittel gelöst und dann in den Lösungsmittelfluß der mobilen Phase einer flüssigkeitschromatographischen Trennsäule eingebracht wird, daß die mobile Phase mit den PAK über eine stationäre Trennphase geleitet wird, auf deren silanol- oder silanolgruppenhaltiger Trägeroberfläche Calixaren in kovalenter Bindung aufgebracht ist, daß die starke Wechselwirkung zwischen dem Calixaren und den verschiedenen Molekülen der PAK unterschiedliche Strömungsgeschwindigkeiten der Moleküle der PAK in der mobilen Phase erzeugt und dadurch die Moleküle der PAK zu unterschiedlichen Zeiten die stationäre Trennphase zeitlich und räumlich getrennt wieder verlassen und der Austritt der Moleküle der PAK aus der stationären Trennphase durch ein Detektorsystem registriert wird.

## Claims

1. A method of separating polycyclic aromatic hydrocarbons (PAHs) by means of liquid chromatography, wherein the sample comprising various PAHs is dissolved in a solvent and is then introduced into the solvent flow of the mobile phase of a liquid chromatographic separation column, the mobile phase comprising the PAHs flows over a stationary separation phase on whose silanol-containing or silanol group-containing surface calixarene is covalently bound, the strong interaction between the calixarene and the different molecules of the PAHs generates different flow velocities of the molecules of the PAHs in the mobile phase so that the molecules of the PAHs leave separated temporally and spatially the stationary separation phase again at different times, and the exit of the molecules of the PAHs from the stationary separation phase is recorded by means of a detector system.

## Revendications

1. Procédé en vue de la séparation d'hydrocarbures aromatiques polycycliques (HAPC) grâce à la chromatographie en phase liquide, **caractérisé en ce que** l'échantillon de substance provenant de divers HAPC est dissous dans un solvant et est alors introduit dans le courant de solvant de la phase mobile d'une colonne de séparation de chromatographie en phase liquide, **en ce que** la phase mobile est conduite avec les HAPC sur une phase de séparation stationnaire, sur la surface support, contenant du silanol ou des groupements de silanol, de laquelle le calixarène est appliqué en liaison covalente, **en ce que** l'interaction forte entre le calixarène et les différentes molécules des HAPC produit des vitesses d'écoulement diverses des molécules de HAPC dans la phase mobile et **en ce que**, de ce fait, les molécules des HAPC quittent à nouveau, à des moments différents, la phase de séparation stationnaire, les molécules étant séparées dans le temps et dans l'espace, et **en ce que** la sortie des molécules de HAPC hors de la phase stationnaire est enregistrée par un système de détection.
